# EUROPÄISCHE PATENTSCHRIFT

(11) **EP 1 306 412 B1**
(45) Veröffentlichungstag und Bekanntmachung des Hinweises auf die Patenterteilung: **02.05.2012**
(21) Anmeldenummer: 02022391.3
(22) Anmeldetag: 10.10.2002
(51) Int. Cl.: C09C 1/00, C09D 5/36

(54) **Farbige Interferenzpigmente**
Coloured interference pigments
Pigments d'interférence colorés

(30) Priorität: 24.10.2001 DE 10151844
(43) Veröffentlichungstag der Anmeldung: 02.05.2003
(73) Patentinhaber: Merck Patent GmbH, 64293 Darmstadt (DE)
(72) Erfinder: Heider, Lilia, Dr., 64560 Riedstadt (DE); Loch, Manuela, 55627 Merxheim (DE); Schupp, Nicole, 64401 Gross-Bieberau (DE); Kniess, Helge Bettina, 64331 Weiterstadt (DE)

(56) Entgegenhaltungen:
- DE-A- 19 618 569
- DE-A- 19 746 067
- DE-A- 19 817 286
- DE-A- 19 836 810
- DE-A- 19 941 607
- DE-A- 19 951 871

## Beschreibung

Die vorliegende Erfindung betrifft farbige Interferenzpigmente auf der Basis von mehrfach beschichteten plättchenförmigen Substraten.

Glanz- oder Effektpigmente werden in vielen Bereichen der Technik eingesetzt, insbesondere im Bereich der Autolacke, der dekorativen Beschichtung, im Kunststoff, in Farben, Druckfarben sowie in kosmetischen Formulierungen.

Glanzpigmente auf Basis transparenter plättchenförmiger Substrate, die keinen "harten" metallischen Glanz aufweisen, sind Gegenstand der WO 93/12182. Glimmerplättchen werden mit einer hochbrechenden Metalloxidschicht, wie z.B. TiO₂, und einer nicht selektiv absorbierenden Schicht belegt. Diese Pigmente zeigen in Abhängigkeit von der TiO₂-Schichtdicke in der Aufsicht eine bestimmte Interferenzfarbe, die mit schräger werdendem Blickwinkel zunehmend schwächer wird und schließlich nach grau bzw. schwarz abkippt. Die Interferenzfarbe ändert sich nicht, aber es ist eine Abnahme der Farbsättigung festzustellen.

Aus der EP 0 753 545 A2 sind goniochromatische Glanzpigmente auf Basis von mehrfach beschichteten, hochbrechenden, für sichtbares Licht zumindest teilweise durchlässigen, nichtmetallischen, plättchenförmigen Substraten bekannt, die mindestens ein Schichtpaket aus einer farblosen niedrigbrechenden und einer reflektierenden, selektiv oder nicht selektiv absorbierenden Beschichtung aufweisen.

Die aus dem Stand der Technik bekannten Mehrschichtpigmente sind teilweise aus nicht oder wenig lichtdurchlässigen Schichtmaterialien aufgebaut und daher in der Anwendung nur sehr beschränkt mit Absorptionspigmenten zu kombinieren. Die Interferenzfarbe dieser Pigmente ist darüber hinaus sehr stark vom Betrachtungswinkel abhängig, was in der Mehrzahl der Anwendungen nicht erwünscht ist. Weiterhin sind diese Pigmente zum Teil sehr schwer herstellbar bzw. reproduzierbar.

Die Mehrschichtpigmente aus der DE 198 17 286 A, DE 197 46 067 A, DE 198 36 810 A und DE 199 51 871 A besitzen auf dem plättchenförmigen Substrat keine definierte Absorption gefolgt von einem Interferenzsystem, wie in Anspruch 1 definiert.

Aufgabe der vorliegenden Erfindung ist es farbige Mehrschichtpigmente mit einer hohen Farbstärke zur Verfügung zu stellen, die keine augeprägte Goniochromatizität besitzen, sich durch ihre vorteilhaften Anwendungseigenschaften auszeichnen und gleichzeitig auf einfache Art und Weise hergestellt werden können.

Überraschenderweise wurden nun farbige Interferenzpigmente auf Basis von mehrfach beschichteten plättchenförmigen Substraten gefunden, auf denen zuerst eine definierte Absorption erzeugt wird und anschließend ein Interferenzsystem definiert wird, Diese Kombination der Absorption und der Interferenz auf einem plättchenförmigen Substrat fährt zu einem farbigen Pigment mit einer hohen Farbreinheit der Interferenzfarbe und einem starken Glanz.

Die erfindungsgemäßen Pigmente zeichnen sich gegenüber den Pigmenten aus dem Stand der Technik durch ihre außerordentlich hohe Buntheit C, ihr hohes Deckvermögen, ihre hohe Farbreinheit der Interferenzfarbe und eine hohe Brillianz aus. Im Gegensatz zu den goniochromatischen Pigmenten aus dem Stand der Technik zeigen sie keine Winkelabhängigkeit der Interferenzfarbe.

Gegenstand der Erfindung sind somit farbige Interferenzpigmente auf der Basis von mehrfach beschichteten plättchenförmigen Substraten gemäß Anspruch 1.

Gegenstand der Erfindung ist weiterhin die Verwendung der erfindungsgemäßen Interferenzpigmente in Farben, Lacken, Druckfarben, Kunststoffen, keramischen Materialien, Gläsern und zur Lasermarkierung. Aufgrund der hohen Farbkraft sind die erfindungsgemäßen Pigmente insbesondere für die dekorative Kosmetik geeignet. Weiterhin sind die erfindungsgemäßen Pigmente auch zur Herstellung von Pigmentpräparationen sowie zur Herstellung von Trockenpräparaten, wie z.B. Granulate, Chips, Pellets, Briketts, etc., geeignet. Die Trockenpräparate sind insbesondere für Druckfarben, Lacke und kosmetische Formulierungen geeignet

Geeignete Basissubstrate für die erfindungsgemäßen farbigen Pigmente sind transparente plättchenförmige Substrate ausgewählt aus der Gruppe natürlicher oder synthetischer Glimmer, BiOCl, Glas-, Al₂O₃-, SiO₂- oder TiO₂-Plättchen. Bevorzugte Substrate sind Schichtsilikate. Es können auch Gemische unterschiedlicher Substrate eingesetzt werden. Besonders bevorzugte Substratgemische bestehen aus

Glimmerplättchen + SiO₂-Plättchen

Glimmerplättchen + Al₂O₃-Plättchen

Glimmerplättchen + Glas-Plättchen

Glimmerplättchen + TiO₂-Plättchen

Glimmerplättchen + Oxinitrid-Plättchen

Glimmerplättchen + Nitrid-Plättchen

Glimmerplättchen + Fischsilber

Glimmerplättchen + Graphitplättchen

Glimmerplättchen + BiOCl

SiO₂- Plättchen + Al₂O₃-Plättchen

Glasplättchen + SiO₂-Plättchen

Die Größe der Basissubstrate ist an sich nicht kritisch und kann auf den jeweiligen Anwendungszweck abgestimmt werden. In der Regel haben die plättchenförmigen Substrate eine Dicke zwischen 0,05 und 1,5 µm, insbesondere zwischen 0,1 und 1 µm. Die Ausdehnung in den beiden anderen Bereichen beträgt üblicherweise zwischen 1 und 250 µm, vorzugsweise zwischen 2 und 200 µm, und insbesondere zwischen 5 und 60 µm. Es können auch Substrate unterschiedlicher Partikelgrößen eingesetzt werden. Besonders bevorzugt ist ein Gemisch aus Glimmerfraktionen von N-Glimmer (10-60 µm) und F-Glimmer (5-20 µm). Weiterhin bevorzugt sind N- und S-Fraktionen (10-130 µm) und F- und S-Fraktionen (5-130 µm).

Die Dicke der einzelnen Schichten auf dem Basissubstrat ist wesentlich für die optischen Eigenschaften des Pigments. Insbesondere die Schicht (A) beeinflusst wesentlich die Farbeigenschaften.

Die erfindungsgemäßen Interferenzpigmente weisen eine Absorptionsschicht (A) und ein Interferenzsystem (B, C, D) auf, letzteres bestehend aus alternierenden hochbrechenden (B, D) und niedrigbrechenden (C) Schichten. Die hochbrechenden Schichten (B) und (D) besitzen einen Brechungsindex von n > 1,8, vorzugsweise von n ≥ 2,0.

Die Schicht (A) besteht vorzugsweise aus farbigen Oxiden, Sulfiden, Telluriden, Seleniden bzw. Mischsystemen dieser Anionen und aus den Elementen der Hauptgruppen 2-5 und der Nebengruppen 1, 2, 4-8, ferner den Lanthaniden und Actiniden. Insbesondere bevorzugt besteht die Schicht (A) aus Fe₂O₃, Fe₃O₄, Ce₂O₃, Cr₂O₃, Ti-Suboxide (TiO₂ teilweise reduziert mit Oxidationszahlen von < 4 bis 2 und niedere Oxide wie Ti₃O₅, Ti₂O₃ bis zu TiO), Titanoxynitride sowie Titannitrid, Molybdänoxide, CoO, Co₃O₄, VO₂, V₂O₃, NiO, MoS₂, WS₂, V₂O₅, CuO, Cu₂O, Ag₂O, CeO₂, MnO₂, Mn₂O₃, Mn₂O₅ bzw. Gemische oder Kombinationen (Mischoxide) davon. Insbesondere bevorzugt besteht die Schicht (A) aus Fe₂O₃, Fe₃O₄, ferner aus Cr₂O₃, CoO, Co₃O₄, VO₂, V₂O₃, NiO, MoS₂, WS₂, V₂O₅, CuO, CeO₂, Ce₂O₃, MnO₂, Mn₂O₃ und/oder Ag₂O.

Die Schichten (B) und (D) bestehen vorzugsweise aus TiO₂, ZrO₂, SnO₂, ZnO, BiOCI. Besonders bevorzugt handelt es sich bei den Schichten (B) und (D) um eine TiO₂-Schicht. Das TiO₂ kann dabei in der Rutil- oder in der Anatasmodifikation vorliegen, vorzugsweise handelt es sich um Rutil.

Als farblose niedrigbrechende für die Beschichtung (C) geeignete Materialien sind vorzugsweise Metalloxide bzw. die entsprechenden Oxidhydrate, wie z.B. SiO₂, Al₂O₃, AlO(OH), B₂O₃, MgF₂, MgSiO₃, oder ein Gemisch der genannten Metalloxide, geeignet. Vorzugsweise besteht die Schicht (C) aus SiO₂, MgF₂, Al₂O₃ oder deren Gemischen.

Für die optischen Eigenschaften der erfindungsgemäßen Pigmente ist es von Vorteil, wenn die Schicht (A) sehr dünn ist. Die Dicke der Schicht (A) beträgt vorzugsweise 1 bis 100 nm, insbesondere 1 bis 50 nm und besonders bevorzugt 5 bis 20 nm.

Die Dicke der Schichten (B) und (D) betragen vorzugsweise 20 bis 250 nm, insbesondere 25 bis 180 nm und besonders bevorzugt 40 bis 150 nm. Die Schichtdicken der Schichten (B) und (D) können gleich oder verschieden sein. Vorzugsweise besitzen sie ähnliche bzw. gleiche Schichtdicken.

Die Dicke der Schicht (C) beträgt vorzugsweise 20 bis 200 nm, insbesondere 30 bis 180 nm und besonders bevorzugt 40 bis 150 nm.

Durch die Beschichtung der Substrate mit einer Absorptionsschicht (A) und mit einer hochbrechenden Schicht (B), einer niedrigbrechenden Schicht (C), einer weiteren farblosen hochbrechenden Schicht (D) entstehen farbige Interferenzpigmente, deren Farbe, Glanz, Deckvermögen in weiten Grenzen variiert werden können.

Besonders bevorzugte Interferenzpigmente weisen folgende Schichtenfolgen auf:

Substrat + Fe₂O₃ (A) + TiO₂ (B) + SiO₂ (C) + TiO₂ (D)

Substrat + Fe₃O₄ (A) + TiO₂ (B) + SiO₂ (C) + TiO₂ (D)

Substrat + Fe₂O₃ (A) + TiO₂ (B) + Al₂O₃ (C) + TiO₂ (D)

Substrat + Fe₃O₄ (A) + TiO₂ (B) + Al₂O₃ (C) + TiO₂ (D)

Substrat + V₂O₅ (A) + TiO₂ (B) + SiO₂ (C) + TiO₂ (D)

Substrat + MnO₂ (A) + TiO₂ (B) + SiO₂ (C) + TiO₂ (D)

Substrat + MnO₂ (A) + TiO₂ (B) + Al₂O₃ (C) + TiO₂ (D)

Substrat + Ag₂O (A) + TiO₂ (B) + SiO₂ (C) + TiO₂ (D)

Substrat + Ag₂O (A) + TiO₂ (B) + Al₂O₃ (C) + TiO₂ (D)

Substrat + CoO (A) + TiO₂ (B) + SiO₂ (C) + TiO₂ (D)

Substrat + Cr₂O₃ (A) + TiO₂ (B) + SiO₂ (C) + TiO₂ (D)

Substrat + Cr₂O₃ (A) + TiO₂ (B) + Al₂O₃ (C) + TiO₂ (D)

Substrat + Ti-Suboxide (A) + TiO₂ (B) + SiO₂ (C) + TiO₂ (D)

Substrat + Ti-Suboxide (A) + TiO₂ (B) + Al₂O₃ (C) + TiO₂ (D)

Substrat + Fe₂O₃ (A) + ZrO₂ (B) + SiO₂ (C) + ZrO₂ (D)

Substrat + Fe₂O₃ (A) + ZnO (B) + SiO₂ (C) + ZrO₂ (D)

Substrat + Fe₃O₄ (A) + ZrO₂ (B) + SiO₂ (C) + ZrO₂ (D)

Substrat + Fe₃O₄ (A) + ZrO₂ (B) + SiO₂ (C) + TiO₂ (D)

Substrat + Fe₂O₃ (A) + TiO₂ (B) + SiO₂ (C) + ZrO₂ (D)

Die erfindungsgemäßen Pigmente lassen sich leicht herstellen durch die Erzeugung einer Absorptionsschicht und hoch- und niedrigbrechender Interferenzschichten mit genau definierter Dicke und glatter Oberfläche auf den feinteiligen, plättchenförmigen Substraten.

Die Metalloxidschichten werden vorzugsweise nasschemisch aufgebracht, wobei die zur Herstellung von Perlglanzpigmenten entwickelten nasschemischen Beschichtungsverfahren angewendet werden können. Derartige Verfahren sind z.B. beschrieben in DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017 oder auch in weiteren dem Fachmann bekannten Patentdokumenten und sonstigen Publikationen.

Bei der Nassbeschichtung werden die Substratpartikel in Wasser suspendiert und mit einem oder mehreren hydrolysierbaren Metallsalzen bei einem für die Hydrolyse geeigneten pH-Wert versetzt, der so gewählt wird, dass die Metalloxide bzw. Metalloxidhydrate direkt auf den Plättchen ausgefällt werden, ohne dass es zu Nebenfällungen kommt. Der pH-Wert wird üblicherweise durch gleichzeitiges Zudosieren einer Base oder Säure konstant gehalten. Anschließend werden die Pigmente abgetrennt, gewaschen und getrocknet und in einer reduzierten Atmosphäre geglüht, wobei die Glühtemperatur in Hinblick auf die jeweils vorliegende Beschichtung optimiert werden kann. In der Regel liegen die Glühtemperaturen zwischen 250 und 900 °C, vorzugsweise zwischen 450 und 700 °C. Falls gewünscht können die Pigmente nach Aufbringen einzelner Beschichtungen abgetrennt, getrocknet oder geglüht werden, um dann zur Auffällung der weiteren Schichten wieder resuspendiert zu werden.

Weiterhin kann die Beschichtung auch in einem Wirbelbettreaktor durch Gasphasenbeschichtung erfolgen, wobei z.B. die in EP 0 045 851 und EP 0 106 235 zur Herstellung von Perlglanzpigmenten vorgeschlagenen Verfahren entsprechend angewendet werden können.

Die Herstellung der Fe₃O₄-Schicht kann z. B. erfolgen durch Reduktion der Fe₂O₃-Schicht mit Ammoniak, Wasserstoff sowie auch Kohlenwasserstoffen und Kohlenwasserstoff/Ammoniak-Gemischen wie z.B. beschrieben in EP-A-0 332 071, DE 19 51 696.8 und DE 19 51 697.7. Vorzugsweise findet die Reduktion in einer Formiergasatmosphäre (N₂/H₂), insbesondere bei 92% N₂/8% H₂ oder 96% N₂/6% H₂, statt. Die Reduktionstemperatur beträgt vorzugsweise 400 bis 700 °C, insbesondere 500 bis 600 °C.

Der Farbton der Pigmente kann in weiten Grenzen durch unterschiedliche Wahl der Belegungsmengen bzw. der daraus resultierenden Schichtdicken variiert werden. Die Feinabstimmung für einen bestimmten Farbton kann über die reine Mengenwahl hinaus durch visuell oder messtechnisch kontrolliertes Anfahren der gewünschten Farbe erreicht werden.

Zur Erhöhung der Licht-, Wasser- und Wetterstabilität empfiehlt es sich häufig in Abhängigkeit vom Einsatzgebiet, das fertige Pigment einer Nachbeschichtung oder Nachbehandlung zu unterziehen. Als Nachbeschichtungen bzw. Nachbehandlungen kommen beispielsweise die in den DE-PS 22 15 191, DE-OS 31 51 354, DE-OS 32 35 017, DE-A 33 34 598, DE 40 30 727 A1, EP 0 649 886 A2, WO 97/29059, WO 99/57204, U.S. 5,759,255 beschriebenen Verfahren in Frage. Durch diese Nachbeschichtung (Schicht E) wird die chemische Stabilität der Pigmente weiter erhöht oder die Handhabung des Pigments, insbesondere die Einarbeitung in unterschiedliche Medien, erleichtert.

Die erfindungsgemäßen Pigmente sind mit einer Vielzahl von Farbsystemen kompatibel vorzugsweise aus dem Bereich der Lacke, Farben und Druckfarben. Für die Herstellung der Druckfarben ist eine Vielzahl von Bindern, insbesondere wasserlösliche Typen, geeignet, wie sie z.B. von den Firmen BASF, Marabu, Pröll, Sericol, Hartmann, Gebr. Schmidt, Sicpa, Aarberg, Siegberg, GSB-Wahl, Follmann, Ruco oder Coates Screen INKS GmbH vertrieben werden. Die Druckfarben können auf Wasserbasis oder Lösemittelbasis aufgebaut sein. Weiterhin sind die Pigmente auch für die Lasermarkierung von Papier und Kunststoffen, sowie für Anwendungen im Agrarbereich, z.B. für Gewächshausfolien, sowie z. B. für die Farbgebung von Zeltplanen, geeignet.

Es versteht sich von selbst, dass für die verschiedenen Anwendungszwecke die Mehrschichtpigmente auch vorteilhaft in Abmischung mit organischen Farbstoffen, organischen Pigmenten oder anderen Pigmenten, wie z. B. transparenten und deckenden Weiß-, Bunt- und Schwarzpigmenten sowie mit plättchenförmigen Eisenoxiden, organischen Pigmenten, holographischen Pigmenten, LCPs (Liquid Crystal Polymers), und herkömmlichen transparenten, bunten und schwarzen Glanzpigmenten auf der Basis von metalloxidbeschichteten Glimmer- und SiO₂-Plättchen, etc., verwendet werden können. Die Mehrschichtpigmente können in jedem Verhältnis mit handelsüblichen Pigmenten und Füllern gemischt werden.

Die erfindungsgemäßen Pigmente sind weiterhin geeignet zur Herstellung von fließfähigen Pigmentpräparationen und Trockenpräparaten. Die Pigmentpräparationen und Trockenpräparate zeichnen sich dadurch aus, dass sie ein oder mehrere erfindungsgemäße Pigmente, Bindemittel und optional ein oder mehrere Additive enthalten.

Gegenstand der Erfindung ist somit auch die Verwendung der Pigmente in Formulierungen wie Farben, Druckfarben, Lacken, Kunststoffen, keramischen Materialien, Gläsern, in kosmetischen Formulierungen, zur Lasermarkierung und zur Herstellung von Pigmentpräparationen und Trockenpräparaten.

Die nachfolgenden Beispiele sollen die Erfindung näher erläutern ohne sie jedoch zu beschränken.

### Beispiele

### Beispiel 1 (Glimmer + Fe₃O₄ + TiO₂ + SiO₂ + TiO₂)

100 g Glimmer der Teilchengröße 10-60 µm werden in 1,9 I VE-Wasser suspendiert und unter starkem Rühren auf 80 °C erhitzt. Der pH-Wert der Glimmersuspension wird mit 10 % Salzsäure auf 2,8 eingestellt. Anschließend werden 111,91 g FeCl₃-Lösung (37,30 g FeCl₃ + 74,61 g H₂O) zudosiert, wobei der pH-Wert mit 32%-iger Natronlauge konstant gehalten wird. Die Suspension wird anschließend 15 Minuten gerührt. Zu dieser Suspension werden bei pH=2,1 890,40 g TiCl₄-Lösung (474,88 g TiCl₄ + 415,52 g H₂O) zudosiert. Danach wird der pH-Wert mit Natronlauge (32 %) auf 7,0 eingestellt und bei diesem pH-Wert werden 188,89 g Natron-wasserglas-Lösung (62,96 g Natronwasserglas (w_{SiO2} = 27 %) + 125,93 g H₂O) zudosiert. Der pH-Wert wird dabei mit Salzsäure (10 %) konstant gehalten. Anschließend werden bei pH 2,1 445,2 g TiCl₄-Lösung (237,44 g TiCl₄ + 207,76 g H₂O) zudosiert. Der pH-Wert wird bei der Zugabe der TiCl₄-Lösung jeweils mit NaOH-Lösung (32%) konstant gehalten. Zur Aufarbeitung wird das Pigment abfiltriert, mit VE-Wasser gewaschen, bei 110 °C getrocknet und 45 min bei 575 °C in einer Formiergasatmosphäre (92 % N2 / 8 % H₂ oder 96 % N₂ / 4 % H₂) reduziert.

Man erhält ein brilliantes Pigment mit grüner Interferenzfarbe kombiniert mit schwarzer Absorptionsfarbe.

Die Messung der Lab-Werte erfolgt mit einem Minolta Farbmessgerät CR-300.

| | |
|---|---|
| L-Wert | zwischen 66 und 70 |
| a-Wert | zwischen -21 und -17 |
| b-Wert | zwischen 13 und 17 |
| C-Wert (Farbstärke) | zwischen 23 und 25 |
| h°-Wert (Bunttonwinkel) | zwischen 139 und 142 |

Ein handelsübliches Interferenzpigment Iriodin® Majestic Green (mit Cr₂O₃ beschichtetes Glimmerpigment der Fa. Merck KGaA) zeigt zum Vergleich folgende Lab-Werte:

| | |
|---|---|
| L-Wert | ca. 68 |
| a-Wert | ca. -21 |
| b-Wert | ca. 12 |
| C-Wert (Farbstärke) | ca. 24 |
| h°-Wert (Bunttonwinkel) | ca. 149° |

Das Pigment aus Beispiel 1 zeichnet sich dagegen insbesondere durch die Chromfreiheit bei gleichem oder ähnlichem Farbton aus.

### Beispiel 2 (Glimmer + Fe₂O₃ + TiO₂ + SiO₂ + TiO₂)

100 g Glimmer der Teilchengröße 10-60 µm werden in 1,9 I VE-Wasser suspendiert und unter starkem Rühren auf 80 °C erhitzt. Der pH-Wert der Glimmersuspension wird mit 10 % Salzsäure auf 2,8 eingestellt. Anschließend werden 111,91 g FeCl₃-Lösung (37,30g g FeCl₃ + 74,61 g H₂O) zudosiert, wobei der pH-Wert mit 32%-iger Natronlauge konstant gehalten wird. Die Suspension wird anschließend 15 Minuten gerührt. Zu dieser Suspension werden bei pH=2,1 890,40 g TiCl₄-Lösung (474,88 g TiCl₄ + 415,52 g H₂O) zudosiert. Danach wird der pH-Wert mit Natronlauge (32 %) auf 7,0 eingestellt und bei diesem pH-Wert werden 188,89 g Natron-wasserglas-Lösung (62,96 g Natronwasserglas (w_{SiO2} = 27 %) + 125,93 g H₂O) zudosiert. Der pH-Wert wird dabei mit Salzsäure (10 %) konstant gehalten. Anschließend werden bei pH 2,1 445,2 g TiCl₄-Lösung (237,44 g TiCl₄ + 207,76 g H₂O) zudosiert. Der pH-Wert wird bei der Zugabe der TiCl₄-Lösung jeweils mit NaOH-Lösung (32%) konstant gehalten. Zur Aufarbeitung wird das Pigment abfiltriert, mit VE-Wasser gewaschen, bei 110 °C getrocknet und 0,5 h bei 600 °C unter Normalatmosphäre geglüht. Man erhält ein Pigment mit grüngelber Körperfarbe/Absorptionsfarbe.

Die Messung der Lab-Werte erfolgt mit einem Minolta Farbmessgerät CR-300.

| | |
|---|---|
| L-Wert | zwischen 75 und 78 |
| a-Wert | zwischen -3,5 und -1,5 |
| b-Wert | zwischen 29 und 31 |
| C-Wert (Farbstärke) | zwischen 29 und 31 |
| h°-Wert (Bunttonwinkel) | zwischen 93° und 97° |

### Beispiel 3 (Glimmer + Fe₂O₃ + TiO₂ + SiO₂ + TiO₂)

50 g Glimmer der Teilchengröße 10-60 µm und 50 g Glimmer der Teilchengröße 5-20 µm werden in 1,91 VE-Wasser suspendiert und unter starkem Rühren auf 80 °C erhitzt. Der pH-Wert der Glimmersuspension wird mit 10 % Salzsäure auf 2,8 eingestellt. Anschließend werden 42 g FeCl₃-Lösung (14 g FeCl₃ + 28 g H₂O) zudosiert, wobei der pH-Wert mit 32%-iger Natronlauge konstant gehalten wird. Die Suspension wird anschließend 15 Minuten gerührt. Zu dieser Suspension werden bei pH = 2,1 408,1 g TiCl₄-Lösung (217,6g TiCl₄ + 190,5 g H₂O) zudosiert. Danach wird der pH-Wert mit Natronlauge (32 %) auf 7,0 eingestellt und bei diesem pH-Wert werden 611,1 g Natronwasserglas-Lösung (203,7 g Natronwasserglas (w_{SiO2} = 27 %) + 407,4 g H₂O) zudosiert. Der pH-Wert wird dabei mit Salzsäure (10 %) konstant gehalten. Anschließend werden bei pH 2,1 200,4 g TiCl₄-Lösung (106,9 g TiCl₄ + 93,5 g H₂O) zudosiert. Der pH-Wert wird bei der Zugabe der TiCl₄-Lösung jeweils mit NaOH-Lösung (32 %) konstant gehalten. Zur Aufarbeitung wird das Pigment abfiltriert, mit VE-Wasser gewaschen, bei 110 °C getrocknet und 0,5 h bei 600 °C unter Normalatmosphäre geglüht. Man erhält ein Pigment mit goldfarbener Körperfarbe/Absorptionsfarbe.

Die Messung der Lab-Werte erfolgt mit einem Minolta Farbmessgerät CR-300.

| | |
|---|---|
| L-Wert | zwischen 80 und 84 |
| a-Wert | zwischen 2 und 6 |
| b-Wert | zwischen 38 und 40 |
| C-Wert (Farbstärke) | zwischen 38 und 40 |
| h°-Wert (Bunttonwinkel) | zwischen 81° und 87° |

### Beispiel 4 (Glimmer + Fe₃O₄ + TiO₂ + SiO₂+ TiO₂)

50 g Glimmer der Teilchengröße 10-130 µm und 50 g Glimmer der Teilchengröße 5-20 µm werden in 1,9 VE-Wasser suspendiert und unter starkem Rühren auf 80 °C erhitzt. Der pH-Wert der Glimmersuspension wird mit 10 % Salzsäure auf 2,8 eingestellt. Anschließend werden 97,9 g FeCl₃-Lösung (32,6 g FeCl₃ + 65,3 g H₂O) zudosiert, wobei der pH-Wert mit 32%-iger Natronlauge konstant gehalten wird. Die Suspension wird anschließend 15 Minuten gerührt. Zu dieser Suspension werden bei pH=2,1 742 g TiCl₄-Lösung (395,7 g TiCl₄ + 346,3 g H₂O) zudosiert. Danach wird der pH-Wert mit Natronlauge (32 %) auf 7,0 eingestellt und bei diesem pH-Wert werden 333,3 g Natronwasserglas-Lösung (111,1 g Natronwasserglas (w_{SiO2}= 27 %) + 222,2 g H₂O) zudosiert. Der pH-Wert wird dabei mit Salzsäure (10 %) konstant gehalten. Anschließend werden bei pH 2,1 371g TiCl₄-Lösung (197,9 g TiCl₄ + 173,1 g H₂O) zudosiert. Der pH-Wert wird bei der Zugabe der TiCl₄-Lösung jeweils mit NaOH-Lösung (32 %) konstant gehalten. Zur Aufarbeitung wird das Pigment abfiltriert, mit VE-Wasser gewaschen, bei 110 °C getrocknet und 45 min bei 575 °C in einer Formiergasatmosphäre (92 % N₂/8 % H₂ oder 96 % N₂/4 % H₂) reduziert. Man erhält ein Pigment mit dunkelblauer Körperfarbe.

Die Messung der Lab-Werte erfolgt mit einem Minolta Farbmessgerät CR-300.

| | |
|---|---|
| L-Wert | zwischen 52 und 56 |
| a-Wert | zwischen -3 und 0 |
| b-Wert | zwischen -20 und -16 |
| C-Wert (Farbstärke) | zwischen 20 und 16 |
| h°-Wert (Bunttonwinkel) | zwischen 259° und 270° |

### Anwendungsbeispiele

### Beispiel A: Nagellack

| | |
|---|---|
| 2,00 % | Pigment aus Beispiel 1 (1) |
| 98,00 % | Thixotrope Nagellack-Base 1348 (Toluene, Ethyl acetate, Butyl acetate, Nitrocellulose, Tosylamide/Formaldehyde, Resin, Dibutyl phthalate, Isopropyl Alcohol, Stearalkonium hectorite, Camphor, Acrylates Copolymer, Benzophenone-1 (2) |

### Bezugsquellen:

(1) Merck KGaA
(2) International Lacquers S. A.

### Beispiel B: Lidschatten

| Phase A | |
|---|---|
| 30,00 % | Pigment aus Beispiel 1 (1) |
| 49,50 % | Talc (1) |
| 7,50 % | Potato Starch (Solanum Tuberosum) (2) |
| 2,50 % | Magnesium Stearate (1) |

| Phase B | |
|---|---|
| 9,14 % | Isopropyl stearate (3) |
| 0,53 % | Cetyl palmitate (1) |
| 0,53 % | Ewalin 1751 (Petrolatum) (4) |
| 0,20 % | Fragrance Elegance # 79228 D MF (Parfum) (5) |
| 0,10 % | Propyl-4-hydroxybenzoate (Propylparaben) (1) |

Die Bestandteile der Phase A werden homogen gemischt. Anschließend wird die Pudermischung unter Rühren mit der geschmolzenen Phase B versetzt. Die Puder werden bei 40-50 bar gepresst.

### Bezugsquellen:

(1) Merck KGaA
(2) Südstärke GmbH
(3) Cognis GmbH
(4) H. Erhard Wagner GmbH
(5) Haarmann & Reimer GmbH

### Beispiel C: Lippenstift

| Phase A | |
|---|---|
| 15,00 % | Pigment aus Beispiel 2 (1) |

| Phase B | |
|---|---|
| 8,75 % | Beeswax white (Cera alba) (1) |
| 5,25 % | Paracera C 44 (Copernicia Cerifera (Carnauba wax, Ceresin) (2) |
| 3,50 % | Adeps Lanae (Lanolin) (3) |
| 5,60 % | Isopropyl myristate (4) |
| 2,10 % | Paraffin viscous (Paraffinum Liquidum (Mineral Oil) (1) |
| 0,05 % | OXYNEX® K liquid (PEG-8, Tocopherol, Ascorbyl palmitate, Ascorbic acid, Citric acid) (1) |
| 0,10 % | Propyl-4-hydroxybenzoate (Propylparaben) (1) |
| 59,45 % | Castor Oil (Ricinus Communis) (3) |

| Phase C | |
|---|---|
| 0,20 % | Fragrance Tendresse # 75418C (Parfum) (5) |

Die Bestandteile der Phase B werden auf 75 °C erhitzt und aufgeschmolzen. Die Pigmente der Phase A werden zugegeben und alles wird gut durchrührt. Die Lippenstiftmasse wird dann in der auf 65 °C temperierten Gießapparatur mit dem Parfüm aus Phase C 15 Minuten gerührt. Die homogene Schmelze wird in die auf 55 °C vorgewärmten Gießformen gegossen. Anschließend kühlt man die Formen ab und entfernt die Gießlinge kalt.

### Bezugsquellen:

(1) Merck KGaA
(2) Paramelt
(3) Henry Lamotte GmbH
(4) Cognis GmbH
(5) Haarmann & Reimer GmbH

### Beispiel D: Klares Duschgel

| Phase A | |
|---|---|
| 0,10 % | Pigment aus Beispiel 1 (1) |
| 0,75 % | Keltrol T (Xanthan Gum) (2) |
| 65,15 % | Wasser (Aqua) |

| Phase B | |
|---|---|
| 20,00 % | Plantacare 2000 UP (Decyl Glucoside) (3) |
| 3,60 % | Texapon ASV (Magnesium Oleth Sulfate, Sodium Oleth Sulfate, Magnesium Laureth-8 Sulfate, Sodium Laureth-8 Sulfate, Magnesium Laureth Sulfate, Sodium Laureth Sulfate (3) |
| 0,20 % | Brondidox L (Propylene Glydol, 5-Bromo-5-Nitro-1,3-Dioxane (3) |
| 0,05 % | Fragrance Everest 79658 SB (Parfum) (4) |
| q.s. % | Dye stuff solution |

| Phase C | |
|---|---|
| 0,15 % | Citric acid monohydrate (Citric acid) (1) |
| 10,00 % | Wasser, demineralisiert (Aqua) |

Das Pigment wird in das Wasser der Phase A dispergiert. Zugabe von Keltrol T und sorgfältiges Mischen der Bestandteile. Die Phase B und die Phase C werden unter Rühren zur Phase A gegeben. Man rührt langsam bis sich das homogene Gel gebildet hat. Der pH-Wert wird auf 6,0-6,5 eingestellt.

### Bezugsquellen:

(1) Merck KGaA
(2) Kelco
(3) Cognis GmbH
(4) Haarmann & Reimer GmbH

### Beispiel E: Lidschattengel

| Phase A | |
|---|---|
| 20,00 % | Pigment aus Beispiel 2 (1) |
| 3,00 % | Ronasphere® (Silica) (1) |
| 0,30 % | Carbopol ETD 2001 (Carbomer) (2) |
| q.s. % | Citric acid monohydrate (Citric acid) (1) |
| 60,00 % | Wasser, demineralisiert (Aqua) |

| Phase B | |
|---|---|
| 2,00 % | Glycerol, anhydrous (Glycerin) (1) |
| 1,00 % | Germaben II (Propylene Glycol, Diazolidinyl Urea, Methylparaben, Propylparaben) (3) |
| 0,70 % | Triethanolamine (Trithanolamine) (1) |
| 13,00 % | Wasser, demineralisiert (Aqua) |

Die Bestandteile von-Phase A werden gemischt. Zugabe von einigen Tropfen Zitronensäure zur Erniedrigung der Viskosität und Zugabe von Carbomer unter Rühren. Solange Rühren bis alles dispergiert ist. Lösen der Bestandteile von Phase B und Rühren bis eine homogene Lösung vorliegt. Unter Rühren Zugabe von Phase B zur Phase A und Einstellung des pH-Werts auf 7,7-7,5.

### Bezugsquellen:

(1) Merck KGaA / Rona®
(2) BF Goodrich GmbH
(3) ISP Global Technologies

### Beispiel E: Wimperntusche

| Phase A | |
|---|---|
| 15,00 % | Pigment aus Beispiel 4 (1) |

| Phase B | |
|---|---|
| 8,00 % | Stearic acid (Stearic acid) (1) |
| 6,00 % | Beeswax white (Cera Alba) (1) |
| 4,00 % | Carnauba Wax 2442 L (Copernicia Cerifera) (2) |
| 3,00 % | Eutanol G (Octyldodecanol) (3) |
| 2,00 % | Arlacel 83 (Sorbitan Sesquioleate) (4) |
| 0,10 % | Propyl-4-hydroxybenzoate (Propylparaben) (1) |
| 0,50 % | RonaCare™ Tocopherol acetate (Tocopheryl Acetate) (1) |

| Phase C | |
|---|---|
| 2,30 % | Triethanolamine (1) |
| 8,00 % | Wasser Soluble Shellac SSB 63 (Shellac) (5) |
| 0,25 % | Methyl-4-hydroxybenzoate (Methylparaben) (1) |
| 0,01 % | RonaCare™ Biotin (Biotin) (1) |
| 50,84 % | Wasser, demineralisiert (Aqua) |

Die Bestandteile der Phase B werden auf 80 °C erhitzt und unter Rühren geschmolzen. Shellac von Phase C wird mit Wasser gemischt, auf 75 °C erhitzt und gelöst in den weiteren Bestandteilen von Phase C. Phase C wird bei 75°C zu dem Gemisch aus Phase A und Phase B zugegeben und für 2 Minuten homogenisiert. Die Wimperntusche wird auf Raumtemperatur abgekühlt und auf pH 7,0-7,5 eingestellt.

### Bezugsquellen:

(1) Merck KGaA/ Rona®
(2) Kahl & Co.
(3) Cognis GmbH
(4) Uniqema
(5) Paroxite Ltd.

## Patentansprüche

1. Farbige Interferenzpigmente mit einer Absorptionsschicht (A) und einem Interferenzsystem (B, C, D) auf der Basis von mehrfach beschichteten plättchenförmigen transparenten Substraten ausgewählt aus der Gruppe natürlicher oder synthetischer Glimmer, BiOCl-, Glas-, Al₂O₃-, SiO₂- oder TiO₂-Plättchen oder deren Gemische, **dadurch gekennzeichnet, dass** sich auf dem Substrat die Schichtenfolge (A)-(D) oder (A)-(E) befindet, wobei
(A) eine farbige Beschichtung mit einem Brechungsindex von n > 1,8
(B) eine farblose Beschichtung mit einem Brechungsindex von n 1,8
(C) eine farblose Beschichtung mit einem Brechungsindex von n ≤ 1,8
(D) eine farblose Beschichtung mit einem Brechungsindex von n > 1,8, und
optional
(E) eine äußere Schutzschicht
ist

2. Farbige Interferenzpigmente nach Anspruch 1, **dadurch gekennzeichnet, dass** das Substratgemisch aus Glimmerfraktionen von N-Glimmer (10-60 µm) und F-Glimmer (5-20 µm), N- und S-Fraktionen (10-130 µm) oder F- und S-Fraktionen (5-130 µm) besteht.

3. Farbige Interferenzpigmente nach Anspruch 1 oder 2, **dadurch gekennzeichnet, dass** die Schicht (A) aus Fe₂O₃, Fe₃O₄, Ce₂O₃, Cr₂O₃, Ti-Suboxiden, Titanoxynitriden, Titannitrid, Molybdänoxiden, CoO, Co₃O₄, VO₂, V₂O₃, NiO, MoS₂, WS₂, V₂O₅, CuO, Cu₂O, Ag₂O, CeO₂, MnO₂, Mn₂O₃, Mn₂O₅ oder deren Gemischen (Mischoxide) besteht.

4. Farbige Interferenzpigmente nach einem oder mehreren der Ansprüche 1 bis 3, **dadurch gekennzeichnet, dass** die Schicht (B) aus TiO₂, ZrO₂, ZnO, BiOCl oder deren Gemischen besteht.

5. Farbige Interferenzpigmente nach einem oder mehreren der Ansprüche 1 bis 4, **dadurch gekennzeichnet, dass** die Schicht (C) aus SiO₂, MgF₂, Al₂O₃ oder deren Gemischen besteht.

6. Farbige Interferenzpigmente nach einem oder mehreren der Ansprüche 1 bis 5, **dadurch gekennzeichnet, dass** die Schicht (D) aus TiO₂, ZrO₂, ZnO, BiOCl oder deren Gemischen besteht.

7. Farbige Interferenzpigmente nach einem oder mehreren der Ansprüche 1 bis 6, **dadurch gekennzeichnet, dass** die Schicht (B) und die Schicht (D) dieselbe Zusammensetzung haben.

8. Farbige Interferenzpigmente nach einem oder mehreren der Ansprüche 1 bis 7, **dadurch gekennzeichnet, dass** die hochbrechenden Schichten (B) und (D) einen Brechungsindex von n ≥ 2,0 besitzen.

9. Farbige Interferenzpigmente nach einem oder mehreren der Ansprüche 1 bis 8, **dadurch gekennzeichnet, dass** die Schicht (B) und die Schicht (D) aus TiO₂ bestehen.

10. Farbige Interferenzpigmente nach einem oder mehreren der Ansprüche 1 bis 9, **dadurch gekennzeichnet, dass** sie folgende Schichtenfolgen aufweisen:
Substrat + Fe₂O₃ (A) + TiO₂ (B) + SiO₂ (C) + TiO₂ (D)
Substrat + Fe₃O₄ (A) + TiO₂ (B) + SiO₂ (C) + TiO₂ (D)
Substrat + Fe₂O₃ (A) + TiO₂ (B) + Al₂O₃ (C) + TiO₂ (D)
Substrat + Fe₃O₄ (A) + TiO₂ (B) + Al₂O₃ (C) + TiO₂ (D)
Substrat + V₂O₅ (A) + TiO₂ (B) + SiO₂ (C) + TiO₂ (D)
Substrat + MnO₂ (A) + TiO₂ (B) + SiO₂ (C) + TiO₂ (D)
Substrat + MnO₂ (A) + TiO₂ (B) + Al₂O₃ (C) + TiO₂ (D)
Substrat + Ag₂O (A) + TiO₂ (B) + SiO₂ (C) + TiO₂ (D)
Substrat + Ag₂O (A) + TiO₂ (B) + Al₂O₃ (C) + TiO₂ (D)
Substrat + CoO (A) + TiO₂ (B) + SiO₂ (C) + TiO₂ (D)
Substrat + Cr₂O₃ (A) + TiO₂ (B) + SiO₂ (C) + TiO₂ (D)
Substrat + Cr₂O₃ (A) + TiO₂ (B) + Al₂O₃ (C) + TiO₂ (D)
Substrat + Ti-Suboxide (A) + TiO₂ (B) + SiO₂ (C) + TiO₂ (D)
Substrat + Ti-Suboxide (A) + TiO₂ (B) + Al₂O₃ (C) + TiO₂ (D)
Substrat + Fe₂O₃ (A) + ZrO₂ (B) + SiO₂ (C) + ZrO₂ (D)
Substrat + Fe₂O₃ (A) + ZnO (B) + SiO₂ (C) + ZrO₂ (D)
Substrat + Fe₃O₄ (A) + ZrO₂ (B) + SiO₂ (C) + ZrO₂ (D)
Substrat + Fe₃O₄ (A) + ZrO₂ (B) + SiO₂ (C) + TiO₂ (D)
Substrat + Fe₂O₃ (A) + TiO₂ (B) + SiO₂ (C) + ZrO₂ (D).

11. Farbige Interferenzpigmente nach einem oder mehreren der Ansprüche 1 bis 10, **dadurch gekennzeichnet, dass** die Dicke der Schicht (A) 1 bis 100 nm beträgt.

12. Farbige Interferenzpigmente nach einem oder mehreren der Ansprüche 1 bis 11, **dadurch gekennzeichnet, dass** die Dicke der Schicht (B) 20 bis 250 nm beträgt.

13. Farbige Interferenzpigmente nach einem oder mehreren der Ansprüche 1 bis 12, **dadurch gekennzeichnet, dass** die Dicke der Schicht (C) 20 bis 200 nm beträgt.

14. Farbige Interferenzpigmente nach einem oder mehreren der Ansprüche 1 bis 13, **dadurch gekennzeichnet, dass** die Dicke der Schicht (D) 20 bis 250 nm beträgt.

15. Farbige Interferenzpigmente nach einem oder mehreren der Ansprüche 1 bis 14, **dadurch gekennzeichnet, dass** das TiO₂ in der Rutil- oder in der Anatasmodifikation vorliegt.

16. Verfahren zur Herstellung der farbigen Interferenzpigmente nach einem oder mehreren der Ansprüche 1 bis 15, **dadurch gekennzeichnet, dass** die Beschichtung der plättchenförmigen Substrate nasschemisch durch hydrolytische Zersetzung von Metallsalzen in wässrigem Medium, durch CVD- oder PVD-Verfahren erfolgt.

17. Verfahren nach Anspruch 16, **dadurch gekennzeichnet, dass** die Herstellung der Fe₃O₄-Schicht durch Reduktion der Fe₂O₃-Schicht mit Ammoniak, Wasserstoff sowie Kohlenwasserstoffen und Kohlenwasserstoff/Ammoniak-Gemischen erfolgt.

18. Verwendung der farbigen Interferenzpigmente nach einem oder mehreren der Ansprüche 1 bis 15 in Farben, Lacken, Druckfarben, Kunststoffen, keramischen Materialien, Gläsern, in kosmetischen Formulierungen, zur Lasermarkierung und zur Herstellung von Pigmentpräparationen und Trockenpräparaten.

19. Verwendung der farbigen Interferenzpigmente nach Anspruch 18 in Pigmentpräparationen enthaltend ein oder mehrere Bindemittel und optional ein oder mehrere Additive.

20. Verwendung der farbigen Interferenzpigmente nach Anspruch 18 in Trockenpräparaten wie Pellets, Granulate, Chips, Briketts.

## Claims

1. Coloured interference pigments having an absorption layer (A) and an interference system (B, C, D) based on multicoated, flake-form, transparent substrates selected from the group natural or synthetic mica, BiOCl flakes, glass flakes, Al₂O₃ flakes, SiO₂ flakes or TiO₂ flakes, or mixtures thereof, **characterised in that** the layer sequence (A)-(D) or (A)-(E) is located on the substrate, where
(A) is a coloured coating having a refractive index of n > 1.8,
(B) is a colourless coating having a refractive index of n > 1.8,
(C) is a colourless coating having a refractive index of n ≤ 1.8,
(D) is a colourless coating having a refractive index of n > 1.8, and
optionally
(E) is an outer protective layer.

2. Coloured interference pigments according to Claim 1, **characterised in that** the substrate mixture consists of mica fractions of N mica (10-60 µm) and F mica (5-20 µm), N and S fractions (10-130 µm) or F and S fractions (5-130 µm).

3. Coloured interference pigments according to Claim 1 or 2, **characterised in that** layer (A) consists of Fe₂O₃, Fe₃O₄, Ce₂O₃, Cr₂O₃, Ti suboxides, titanium oxynitrides, titanium nitride, molybdenum oxides, CoO, Co₃O₄, VO₂, V₂O₃, NiO, MoS₂, WS₂, V₂O₅, CuO, Cu₂O, Ag₂O, CeO₂, MnO₂, Mn₂O₃, Mn₂O₅ or mixtures thereof (mixed oxides).

4. Coloured interference pigments according to one or more of Claims 1 to 3, **characterised in that** layer (B) consists of TiO₂, ZrO₂, ZnO, BiOCl or mixtures thereof.

5. Coloured interference pigments according to one or more of Claims 1 to 4, **characterised in that** layer (C) consists of SiO₂, MgF₂, Al₂O₃ or mixtures thereof.

6. Coloured interference pigments according to one or more of Claims 1 to 5, **characterised in that** layer (D) consists of TiO₂, ZrO₂, ZnO, BiOCl or mixtures thereof.

7. Coloured interference pigments according to one or more of Claims 1 to 6, **characterised in that** layer (B) and layer (D) have the same composition.

8. Coloured interference pigments according to one or more of Claims 1 to 7, **characterised in that** the high-refractive-index layers (B) and (D) have a refractive index of n ≥ 2.0.

9. Coloured interference pigments according to one or more of Claims 1 to 8, **characterised in that** layer (B) and layer (D) consist of TiO₂.

10. Coloured interference pigments according to one or more of Claims 1 to 9, **characterised in that** they have the following layer sequences:
substrate + Fe₂O₃ (A) + TiO₂ (B) + SiO₂ (C) + TiO₂ (D)
substrate + Fe₃O₄ (A) + TiO₂ (B) + SiO₂ (C) + TiO₂ (D)
substrate + Fe₂O₃ (A) + TiO₂ (B) + Al₂O₃ (C) + TiO₂ (D)
substrate + Fe₃O₄ (A) + TiO₂ (B) + Al₂O₃ (C) + TiO₂ (D)
substrate + V₂O₅ (A) + TiO₂ (B) + SiO₂ (C) + TiO₂ (D)
substrate + MnO₂ (A) + TiO₂ (B) + SiO₂ (C) + TiO₂ (D)
substrate + MnO₂ (A) + TiO₂ (B) + Al₂O₃ (C) + TiO₂ (D)
substrate + Ag₂O (A) + TiO₂ (B) + SiO₂ (C) + TiO₂ (D)
substrate + Ag₂O (A) + TiO₂ (B) + Al₂O₃ (C) + TiO₂ (D)
substrate + CoO (A) + TiO₂ (B) + SiO₂ (C) + TiO₂ (D)
substrate + Cr₂O₃ (A) + TiO₂ (B) + SiO₂ (C) + TiO₂ (D)
substrate + Cr₂O₃ (A) + TiO₂ (B) + Al₂O₃ (C) + TiO₂ (D)
substrate + Ti suboxides (A) + TiO₂ (B) + SiO₂ (C) + TiO₂ (D)
substrate + Ti suboxides (A) + TiO₂ (B) + Al₂O₃ (C) + TiO₂ (D)
substrate + Fe₂O₃ (A) + ZrO₂ (B) + SiO₂ (C) + ZrO₂ (D)
substrate + Fe₂O₃ (A) + ZnO (B) + SiO₂ (C) + ZrO₂ (D)
substrate + Fe₃O₄ (A) + ZrO₂ (B) + SiO₂ (C) + ZrO₂ (D)
substrate + Fe₃O₄ (A) + ZrO₂ (B) + SiO₂ (C) + TiO₂ (D)
substrate + Fe₂O₃ (A) + TiO₂ (B) + SiO₂ (C) + ZrO₂ (D).

11. Coloured interference pigments according to one or more of Claims 1 to 10, **characterised in that** the thickness of layer (A) is 1 to 100 nm.

12. Coloured interference pigments according to one or more of Claims 1 to 11, **characterised in that** the thickness of layer (B) is 20 to 250 nm.

13. Coloured interference pigments according to one or more of Claims 1 to 12, **characterised in that** the thickness of layer (C) is 20 to 200 nm.

14. Coloured interference pigments according to one or more of Claims 1 to 13, **characterised in that** the thickness of layer (D) is 20 to 250 nm.

15. Coloured interference pigments according to one or more of Claims 1 to 14, **characterised in that** the TiO₂ is in the rutile or anatase modification.

16. Process for the preparation of the coloured interference pigments according to one or more of Claims 1 to 15, **characterised in that** the coating of the flake-form substrates is carried out by wet-chemical methods by hydrolytic decomposition of metal salts in aqueous medium or by CVD or PVD methods.

17. Process according to Claim 16, **characterised in that** the Fe₃O₄ layer is produced by reduction of the Fe₂O₃ layer using ammonia, hydrogen and hydrocarbons and hydrocarbon/ammonia mixtures.

18. Use of the coloured interference pigments according to one or more of Claims 1 to 15 in paints, coatings, printing inks, plastics, ceramic materials, glasses, in cosmetic formulations, for laser marking and for the preparation of pigment compositions and dry preparations.

19. Use of the coloured interference pigments according to Claim 18 in pigment compositions comprising one or more binders and optionally one or more additives.

20. Use of the coloured interference pigments according to Claim 18 in dry preparations, such as pellets, granules, chips, briquettes.

## Revendications

1. Pigments d'interférence colorés ayant une couche d'absorption (A) et un système d'interférence (B, C, D) à base de substrats transparents, sous forme de paillettes et multicouches, choisis dans le groupe constitué par le mica naturel ou synthétique, les paillettes de BiOCl, les paillettes de verre, les paillettes d'Al₂O₃, les paillettes de SiO₂ ou les paillettes de TiO₂, ou des mélanges de ceux-ci, **caractérisés en ce que** la séquence de couches (A)-(D) ou (A)-(E) est située sur le substrat, où
(A) est un revêtement coloré ayant un indice de réfraction n > 1,8,
(B) est un revêtement incolore ayant un indice de réfraction n > 1,8,
(C) est un revêtement incolore ayant un indice de réfraction n ≤ 1,8,
(D) est un revêtement incolore ayant un indice de réfraction n > 1,8, et
éventuellement
(E) est une couche protectrice externe.

2. Pigments d'interférence colorés selon la revendication 1, **caractérisés en ce que** le mélange de substrats est constitué de fractions de mica de mica N (10-60 µm) et de mica F (5-20 µm), de fractions N et S (10-130 µm) ou de fractions F et S (5-130 µm).

3. Pigments d'interférence colorés selon la revendication 1 ou 2, **caractérisés en ce que** la couche (A) est constituée de Fe₂O₃, Fe₃O₄, Ce₂O₃, Cr₂O₃, de sous-oxydes de Ti, d'oxynitrures de titane, de nitrure de titane, d'oxydes de molybdène, de CoO, Co₃O₄, VO₂, V₂O₃, NiO, MoS₂, WS₂, V₂O₅, CuO, Cu₂O, Ag₂O, CeO₂, MnO₂, Mn₂O₃, Mn₂O₅ ou de mélanges de ceux-ci (oxydes mixtes).

4. Pigments d'interférence colorés selon l'une ou plusieurs parmi les revendications 1 à 3, **caractérisés en ce que** la couche (B) est constituée de TiO₂, ZrO₂, ZnO, BiOCl ou de mélanges de ceux-ci.

5. Pigments d'interférence colorés selon l'une ou plusieurs parmi les revendications 1 à 4, **caractérisés en ce que** la couche (C) est constituée de SiO₂, MgF₂, Al₂O₃ ou de mélanges de ceux-ci.

6. Pigments d'interférence colorés selon l'une ou plusieurs parmi les revendications 1 à 5, **caractérisés en ce que** la couche (D) est constituée de TiO₂, ZrO₂, ZnO, BiOCl ou de mélanges de ceux-ci.

7. Pigments d'interférence colorés selon l'une ou plusieurs parmi les revendications 1 à 6, **caractérisés en ce que** la couche (B) et la couche (D) possèdent la même composition.

8. Pigments d'interférence colorés selon l'une ou plusieurs parmi les revendications 1 à 7, **caractérisés en ce que** les couches (B) et (D) d'indice de réfraction élevé possèdent un indice de réfraction n ≥ 2,0.

9. Pigments d'interférence colorés selon l'une ou plusieurs parmi les revendications 1 à 8, **caractérisés en ce que** la couche (B) et la couche (D) sont constituées de TiO₂.

10. Pigments d'interférence colorés selon l'une ou plusieurs parmi les revendications 1 à 9, **caractérisés en ce qu'**ils possèdent les séquences de couches suivantes :
substrat + Fe₂O₃ (A) + TiO₂ (B) + SiO₂ (C) + TiO₂ (D)
substrat + Fe₃O₄ (A) + TiO₂ (B) + SiO₂ (C) + TiO₂ (D)
substrat + Fe₂O₃ (A) + TiO₂ (B) + Al₂O₃ (C) + TiO₂ (D)
substrat + Fe₃O₄ (A) + TiO₂ (B) + Al₂O₃ (C) + TiO₂ (D)
substrat + V₂O₅ (A) + TiO₂ (B) + SiO₂ (C) + TiO₂ (D)
substrat + MnO₂ (A) + TiO₂ (B) + SiO₂ (C) + TiO₂ (D)
substrat + MnO₂ (A) + TiO₂ (B) + Al₂O₃ (C) + TiO₂ (D)
substrat + Ag₂O (A) + TiO₂ (B) + SiO₂ (C) + TiO₂ (D)
substrat + Ag₂O (A) + TiO₂ (B) + Al₂O₃ (C) + TiO₂ (D)
substrat + CoO (A) + TiO₂ (B) + SiO₂ (C) + TiO₂ (D)
substrat + Cr₂O₃ (A) + TiO₂ (B) + SiO₂ (C) + TiO₂ (D)
substrat + Cr₂O₃ (A) + TiO₂ (B) + Al₂O₃ (C) + TiO₂ (D)
substrat + sous-oxydes de Ti (A) + TiO₂ (B) + SiO₂ (C) + TiO₂ (D)
substrat + sous-oxydes de Ti (A) + TiO₂ (B) + Al₂O₃ (C) + TiO₂ (D)
substrat + Fe₂O₃ (A) + ZrO₂ (B) + SiO₂ (C) + ZrO₂ (D)
substrat + Fe₂O₃ (A) + ZnO (B) + SiO₂ (C) + ZrO₂ (D)
substrat + Fe₃O₄ (A) + ZrO₂ (B) + SiO₂ (C) + ZrO₂ (D)
substrat + Fe₃O₄ (A) + ZrO₂ (B) + SiO₂ (C) + TiO₂ (D)
substrat + Fe₂O₃ (A) + TiO₂ (B) + SiO₂ (C) + ZrO₂ (D).

11. Pigments d'interférence colorés selon l'une ou plusieurs parmi les revendications 1 à 10, **caractérisés en ce que** l'épaisseur de la couche (A) va de 1 à 100 nm.

12. Pigments d'interférence colorés selon l'une ou plusieurs parmi les revendications 1 à 11, **caractérisés en ce que** l'épaisseur de la couche (B) va de 20 à 250 nm.

13. Pigments d'interférence colorés selon l'une ou plusieurs parmi les revendications 1 à 12, **caractérisés en ce que** l'épaisseur de la couche (C) va de 20 à 200 nm.

14. Pigments d'interférence colorés selon l'une ou plusieurs parmi les revendications 1 à 13, **caractérisés en ce que** l'épaisseur de la couche (D) va de 20 à 250 nm.

15. Pigments d'interférence colorés selon l'une ou plusieurs parmi les revendications 1 à 14, **caractérisés en ce que** le TiO₂ se trouve selon la modification rutile ou anatase.

16. Procédé de préparation des pigments d'interférence colorés selon l'une ou plusieurs parmi les revendications 1 à 15, **caractérisé en ce que** le revêtement des substrats sous forme de paillettes est effectué selon des méthodes de chimie par voie humide par décomposition hydrolytique de sels métalliques en milieu aqueux ou selon des méthodes de dépôt chimique ou physique en phase vapeur.

17. Procédé selon la revendication 16, **caractérisé en ce que** la couche de Fe₃O₄ est produite par réduction de la couche de Fe₂O₃ en utilisant de l'ammoniac, de l'hydrogène et des hydrocarbures et des mélanges hydrocarbures/ammoniac.

18. Utilisation des pigments d'interférence colorés selon l'une ou plusieurs parmi les revendications 1 à 15 dans des peintures, des revêtements, des encres d'impression, des plastiques, des matériaux en céramique, des verres, dans des formulations cosmétiques, pour un marquage laser et pour la préparation de compositions de pigments et de préparations sèches.

19. Utilisation des pigments d'interférence colorés selon la revendication 18 dans des compositions de pigments comprenant un ou plusieurs liants et éventuellement un ou plusieurs additifs.

20. Utilisation des pigments d'interférence colorés selon la revendication 18 dans des préparations sèches, telles que des pastilles, des granulés, des copeaux et des briquettes.
